# EUROPEAN PATENT APPLICATION

(11) **EP 3 862 419 A1**
(43) Date of publication of application: **11.08.2021**
(21) Application number: 20156006.7
(22) Date of filing: 07.02.2020
(51) Int. Cl.: C12M 1/42

(54) **NON-CONTACT DEVICE FOR TREATING CELLS**

(71) Applicant: Bühler AG, 9240 Uzwil (CH)
(72) Inventor: ARNULNESAN, Johan, London SW19 3 AL (GB); MATHYS, Alexander, 8044 Zürich (CH); BUCHMANN, Leandro, 8408 Winterthur (CH); GEORGET, Erika, 8041 Zürich (CH); BUENDER, Jana Carmen, 6283 Baldegg (CH); KINSELLA, David, London SW12 9RT (GB)
(74) Representative: Hepp Wenger Ryffel AG

(57) **Abstract**

The present invention relates to a device (1) for treating cells for stimulating cell growth, comprising a unit (2) for generating and emitting electric pulses, and a treatment unit (3) having an inlet (3a) and an outlet (3a), a treatment space (4) being formed inside the treatment unit (3), the treatment space (4) being penetrated by the emitted electric pulses and an electric field resulting therefrom, a cell material entering into the treatment space (4) through the inlet (3a) being moved without contact through the treatment space (4) and the electric field penetrating the treatment space (4) to the outlet (3b).

## Description

The present invention relates to a non-contact device for treating cells used in medical, environmental, food applications, and bio-based industries (including yeast, bacteria, microalgae, as well as plant or animal cells and cell tissue production systems, in particular targeting inactivation, the extraction of bioactive compounds, and/or the stimulation of cell growth and/or cellular compounds.

It is known that prokaryotic and eukaryotic cells are influenced by the action of electric fields. Stimulation of cell growth, as well as cell death, inactivation of microorganisms, or specific extraction of cell constituents can occur, depending on the applied electric field strength (e.g. Buchmann L and Mathys A (2019), Perspective on Pulsed Electric Field Treatment in the Bio-based Industry, Front. Bioeng. Biotechnol. 7:265, doi: 10.3389/fbioe.2019.00265).

EP-2 308 969 B1 describes a PEF (pulsed electric field) method where a cell material suspended in an electrically conductive liquid, the cell material being positioned between two electrodes, by exposure of 1 to 100 electric pulses, such that a voltage increase takes place between the two electrodes of 10% to 90% of a target voltage of the electric pulses within a period of 0.1 to 100 ns, the electric pulses have a pulse duration of 5 ns to 5000 ns, and the electric pulses, upon reaching the target voltage, have an electric field strength of 0.5 kV/cm to 50 kV/cm, showed an accelerated cell proliferation and/or increased cell constituents.

In the device for treating cell material used in EP 2 308 969 B1, an electroporation cuvette having the suspension of cell material described above was continuously pumped through an arrangement of two electrodes and exposed to an electric field in an electrically conductive liquid (paragraph [0020] of EP-2 308 969 B1).

Under these conditions, a flow profile is formed between the wall and the center of the vessel, which may result in deviations with respect to the process conditions over the vessel diameter. This is a problem since influencing the cell proliferation as described above depends on the accurate maintenance of the process conditions. With the device of EP-2 308 969 B1, it is not possible to obtain homogeneous process conditions in the entire reaction vessel and therefore also no homogeneously influenced cell material.

It was the object of the present invention to overcome the above problems of the prior art and to provide a device that overcomes difficulties involved with currently available PEF equipment in order to obtain homogenous and controllable flow and electric field profiles.

The above object is achieved by the present invention as defined in the claims.

In detail, the present invention relates to a device, comprising a unit for generating and emitting electric pulses, and a treatment unit having an inlet and an outlet, wherein a treatment space is formed inside the treatment unit, the treatment space being able to be penetrated by the emitted electric pulses and an electric field resulting therefrom, characterized in that a cell material entering through the inlet into the treatment space can be moved without contact through the treatment space and the electric field penetrating the treatment space to the outlet.
According to the present invention, the device is suitable for medical, environmental, food applications, and bio-based industries (including yeast, bacteria, microalgae, as well as plant or animal cells and cell tissue production systems, in particular targeting inactivation, the extraction of bioactive compounds, and/or the stimulation of cell growth and/or cellular compounds. These applications are described in Buchmann L and Mathys A (2019), Perspective on Pulsed Electric Field Treatment in the Bio-based Industry, Front. Bioeng. Biotechnol. 7:265, doi: 10.3389/fbioe.2019.00265.

According to the present invention, an electrical field is applied to a cell material located in a container while it passes without contact through a treatment space in a treatment unit. As a result, the cell material is subjected to the electric field without interactions with boundary walls, and thus a more homogeneous stimulation of cell growth is achieved.

According to the present invention, a non-contact movement (i.e. "without contact") is understood to mean that the material undergoing movement does not come into direct contact with boundary walls (for example, a pipe or a container) during the movement. A non-contact movement according to the present invention is therefore characterized in that there are no interactions with boundary walls during the interaction and in particular no inhomogeneous flow profile (in the case of the movement of a liquid, suspension or emulsion) is formed.

According to the present invention, basically any material composed of at least one cell, that is, both eukaryotic and prokaryotic cells, can be used as the cell material to be treated. The cell material can be unicellular or multicellular organisms. Examples would be bacteria, yeasts, microalgae, plant cells, and fungal cells or their spores, mycelia, seeds or seedlings and somatic animal cells. Furthermore, multicellular tissues such as meristems in plants and epithelial or connective tissue in humans or animals can be treated.

The cell material is usually (but not necessarily) isolated, purified and/or sterilized in a known manner before being treated according to the present invention. Preferably, the cell material can already be propagated in a known manner in suitable and known culture media to a desired degree before the treatment according to the present invention.

The cell material is preferably suspended in an electrically conductive liquid prior to the treatment according to the present invention. Electrically conductive liquids are well known. According to the present invention, it is necessary to use electrically conductive liquids which have no adverse effects on cell viability, that is, in particular, are non-toxic. According to the present invention, water is preferably used as the electrically conductive liquid, wherein the water can be adjusted to a desired pH value by means of suitable and known additives. According to the present invention, a pH value in the range of 6.0 to 14.0, preferably 7 to 12 is preferred.

According to the present invention, the suspensions described above can be prepared in a conventional manner and stored until treatment. However, the suspensions can also be provided immediately before the treatment according to the present invention.

The cell material or a suspension containing the cell material is passed through the inlet of a treatment unit into a treatment space which is located inside the treatment unit.

The inlet of the treatment unit is preferably connected to a device in which the cell material or a suspension containing the cell material is located. For example, conventional pipelines can be provided for this connection.

Preferably, a shut-off unit such as a valve or a lock is provided in or at the inlet or alternatively in the connection described above in order to be able to control the introduction of the cell material or the suspension containing the cell material into the treatment unit.

According to a preferred embodiment of the present invention, the inlet comprises a nozzle or the inlet constitutes a nozzle. It is possible to use nozzles which can conventionally be used for introducing a medium into a container or defined space. Such nozzles are known.

With this embodiment, the speed at which the cell material or a suspension containing the cell material is introduced into the treatment unit can be adjusted in a targeted manner. The residence time of the cell material in the electric field applied to the treatment space of the treatment unit can thereby be adjusted, in combination with the length of the treatment unit.

The treatment unit into which the cell material to be treated is introduced through the inlet described above is limited with respect to its shape, structure and dimensions only in that an applied electric field as described below can sufficiently penetrate the treatment unit to achieve the desired treatment of the cell material in the treatment unit. According to the present invention, the treatment unit is preferably a container made of a metallic material or a plastic material (such as polyethylene terephthalate).

The treatment unit can have any geometric shape which allows a non-contact passage of the cell material. Preferably, the treatment unit is a cylindrical body.

A treatment space is provided within the treatment unit, through which treatment space the cell material passes and is thereby treated with an electric field.

The present invention is characterized by a non-contact passage of the cell material through the treatment space and the electric field penetrating the treatment space to an outlet of the treatment unit. The treatment space therefore has a suitable size to allow the desired non-contact passage of the cell material. According to a preferred embodiment, the treatment unit is a hollow body, particularly preferably a cylindrical hollow body, the interior of which constitutes the treatment space.

The inlet and the outlet are arranged on the treatment unit such that the above non-contact passage of the cell material can take place through the treatment space.

According to a preferred embodiment of the present invention, the passage of the cell material through the treatment space takes place under the action of the gravitational force. In this case, the inlet and outlet are to be arranged relative to one another such that the cell material entering through the inlet passes to the outlet through the action of the gravitational force and can escape therefrom out of the treatment unit. Particularly preferred is an arrangement of inlet and outlet relative to each other such that the outlet is arranged below the inlet, so that the outlet is in the fall line of the inlet. In this case, the inlet is preferably arranged in the top surface, preferably the center of the top surface, of the treatment unit and the outlet arranged in the bottom, preferably the center of the bottom, of the treatment unit. Particularly preferred is thus an arrangement of inlet and outlet relative to each other, in which the outlet is located in the fall line of the inlet. According to the present invention, the fall line is the path which a body travels in free fall under the influence of the gravitational force.

An arrangement of the inlet in a side wall of the treatment unit is also conceivable, through which the cell material enters into the treatment space at a suitable speed and passes on a defined path to the outlet.

According to a preferred embodiment of the present invention, a receiving device is provided in the treatment unit, with which receiving device the cell material moved through the treatment space can be supplied to the outlet. The receiving device provides an opening which is widened in relation to the opening of the outlet. For this purpose, the receiving device has an upper opening with a larger area and a lower opening with a smaller area, wherein the size of the areas is related to the respective other opening. According to the present invention, the receiving device is preferably a funnel-shaped unit having circular openings.

Preferably, the receiving device is arranged in the treatment space immediately above the outlet, so that the lower opening of the receiving device is arranged flush with the opening of the outlet, wherein in particular preferably the lower opening of the receiving device and the opening of the outlet have the same area.

According to a preferred embodiment of the present invention, the treatment space is filled with a dielectric material in order to ensure or improve the penetration of the treatment space with the applied electric field. Preferably, the dielectric material is a gas, more preferably air.

As stated above, the cell material passes through the treatment space due to the action of the gravitational force of the earth. However, it is also possible to move the cell material in an alternative way without contact in a defined manner through the treatment space. For example, the device can further have a unit for generating a magnetic field in the treatment space. The defined movement of charged particles with the aid of an applied magnetic field and the units which can be used for generating a magnetic field are known. Also, alternatively the principle of the Vegas fountain or in general of water fountains can be employed, where a bundle of narrow channels (e.g. by specifically designed nozzles allowing for flow control during non-contact passage) is utilized to direct and influence the fluid flow in air.

The cell material is subjected to a treatment by an applied electric field in the treatment space.

Units for generating and emitting electric pulses are well known. According to the present invention, devices which can generate electrical impulses as described below are preferred. Such devices are known. By way of example, cable pulse generators, semiconductor-based pulse generators, or relaxation oscillators can be mentioned.

The generated electric pulses are emitted in a treatment space. This is preferably done by two or more electrodes or plates of a capacitor arranged parallel to each other, which are arranged opposite each another having a distance suitable for the generation of electric pulses. Such arrangements are well known and need not be explained in detail here.

The electric field to be applied to the treatment space must be characterized such that it provides for the desired effect, e.g. that it stimulates the growth of the treated cells. Corresponding electric fields are known from the prior art, for example from EP-2 308 969 B1. According to the present invention, an electric field generated from such electric pulses can be used, such that a voltage increase takes place between the two electrodes or plates of a capacitor of the device of 10% to 90% of a target voltage of the electric pulses within a period of 0.1 to 1000 ns, the electric pulses have a pulse duration of 5 ns to 50000 ns, and the electric pulses, upon reaching the target voltage, have an electric field strength of 0.5 kV/cm to 100 kV/cm.

The treatment unit having the cell material to be treated is arranged according to the present invention in the space between the two electrodes or plates of a capacitor parallel to each other.

The present invention further relates to a method for treating cells for stimulating cell growth, preferably performed in a device as described above, comprising the steps
a) applying an electric field to a treatment space in a treatment unit,
b) introducing cell material through an inlet of the treatment unit into the treatment space,
c) passing of the cell material without contact through the treatment space and the electric field penetrating the treatment space to an outlet of the treatment unit.

The method can be performed as already described above.

As stated above, it is preferred that the cell material is provided as a suspension in a dielectric material in the at least one container.

As stated above, it is preferred that step c) (non-contact passage of the cell material through the treatment space and the electric field penetrating the treatment space to an outlet of the treatment unit) takes place due to gravitational force.

As stated above, it is alternatively possible for step c) (non-contact passage of the cell material through the treatment space and the electric field penetrating the treatment space to an outlet of the treatment unit) takes place by deflection of the cell material in a magnetic field.

As stated above, it is further preferred that an electric field is applied with such electric pulses, so that a voltage increase takes place between the two electrodes or plates of a capacitor of the device of 10% to 90% of a target voltage of the electric pulses within a period of 0.1 to 1000 ns, the electric pulses have a pulse duration of 5 ns to 50000 ns, and the electric pulses, upon reaching the target voltage, have an electric field strength of 0.5 kV/cm to 100 kV/cm.

The present invention further relates to the use of the device according to the present invention described here suitable for medical, environmental, food applications, and bio-based industries (including yeast, bacteria, microalgae, as well as plant or animal cells and cell tissue production systems, in particular targeting inactivation, the extraction of bioactive compounds, and/or the stimulation of cell growth and/or cellular compounds. These applications are described in Buchmann L and Mathys A (2019), Perspective on Pulsed Electric Field Treatment in the Bio-based Industry, Front. Bioeng. Biotechnol. 7:265, doi: 10.3389/fbioe.2019.00265.

The present invention is explained below by way of non-limiting examples and figures. Shown are:

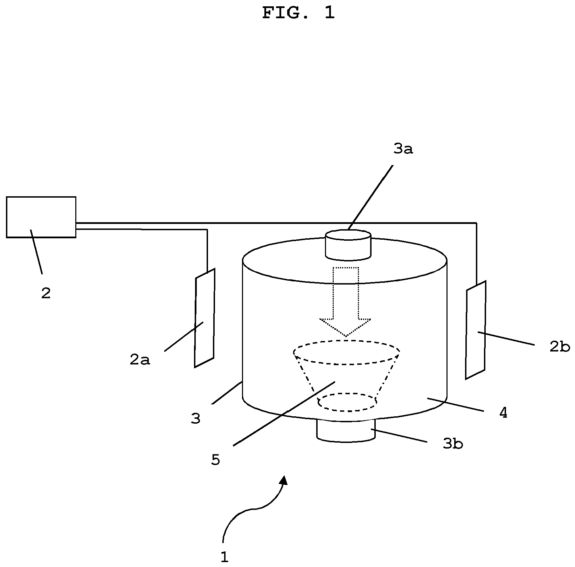

- Fig. 1: a schematic representation of an embodiment of the device of the present invention.

Fig. 1 shows a schematic representation of an embodiment of the device (1) of the present invention for treating cells for stimulating cell growth.

The device has a unit (2) for generating and emitting electric pulses, for example, a pulse generator. The unit (2) is electrically connected to two electrodes (2a, 2b). A here cylindrical treatment unit (3) having a treatment space (4) in its interior is located between the two electrodes (2a, 2b), to which treatment space electric pulses generated by the unit (2) are applied.

An inlet (3a) is arranged on the top surface of the treatment unit (3) according to this embodiment, preferably in the center of the top surface of the treatment unit (3). An outlet (3b) is arranged on the bottom surface of the treatment unit (3) according to this embodiment, preferably in the center of the bottom surface of the treatment unit (3). According to this embodiment, the outlet (3b) is arranged in the fall line of a cell material which enters into the treatment unit (3) through the inlet (3a) and passes through the treatment space (4) due to gravitational force.

A here funnel-shaped receiving device (5) is arranged in the treatment space (4). The receiving device (5) is arranged according to the embodiment shown here in the treatment space (4) immediately above the outlet (3b), so that the lower opening of the receiving device (5) is arranged flush with the (not shown here) opening of the outlet (3b). According to the embodiment shown here, the lower opening of the receiving device (5) and the opening of the outlet (3b) have the same area.

## Claims

1. A device (1), comprising a unit (2) for generating and emitting electric pulses, and a treatment unit (3) having an inlet (3a) and an outlet (3a), wherein a treatment space (4) is formed inside the treatment unit (3), the treatment space (4) being able to be penetrated by the emitted electric pulses and an electric field resulting therefrom, **characterized in that** a cell material entering through the inlet (3a) into the treatment space (4) can be moved without contact through the treatment space (4) and the electric field penetrating the treatment space (4) to the outlet (3b).

2. The device according to claim 1, **characterized in that** a receiving device (5) is provided in the treatment unit (3), with which receiving device (5) the cell material moved through the treatment space (4) can be supplied to the outlet (3b).

3. The device according to claim 2, **characterized in that** the receiving device (5) is a funnel-shaped treatment unit.

4. The device according to any one of claims 1 to 3, **characterized in that** the inlet (3a) comprises a nozzle.

5. The device according to any one of claims 1 to 4, **characterized in that** the treatment space (4) is filled with a dielectric material.

6. The device according to claim 5, **characterized in that** the dielectric material is a gas, preferably air.

7. The device according to any one of claims 1 to 6, **characterized in that** the outlet (3b) is arranged below the inlet (3a), so that the outlet (3b) is located in the fall line of the inlet (3a).

8. The device according to any one of claims 1 to 7, **characterized in that** the device (1) further has a unit for generating a magnetic field in the treatment space (4).

9. The device according to any one of claims 1 to 8, **characterized in that** the unit (2) for generating and emitting electric pulses comprises two or more electrodes (2a, 2b) or plates of a capacitor and can generate electric pulses, so that a voltage increase take place between the two or more electrodes (2a, 2b) of 10% to 90% of a target voltage of the electric pulses within a period of 0.1 to 1000 ns, the electric pulses have a pulse duration of 5 ns to 50000 ns, and the electric pulses, upon reaching the target voltage, have an electric field strength of 0.5 kV/cm to 100 kV/cm.

10. A method for treating cells for targeted inactivation, the extraction of bioactive compounds, and the stimulation of cell growth and/or cellular compounds, preferably performed in a device (1) according to any one of claims 1 to 9, comprising the steps
a) applying an electric field to a treatment space (4) in a treatment unit (3),
b) introducing cell material through an inlet (3a) of the treatment unit (3) into the treatment space (4),
c) passing of the cell material without contact through the treatment space (4) and the electric field penetrating the treatment space (4) to an outlet (3b) of the treatment unit (3).

11. The method according to claim 10, **characterized in that** the cell material is provided as a suspension in a dielectric material.

12. The method according to claim 10 or 11, **characterized in that** the step c) takes place due to gravitational force.

13. The method according to claim 10 or 11, **characterized in that** the step c) takes place by deflecting the cell material in a magnetic field.

14. The method according to any one of claims 10 to 13, **characterized in that** an electric field is applied with such electric pulses, so that a voltage increase takes place between the two electrodes (2a, 2b) or plates of a capacitor of 10% to 90% of a target voltage of the electric pulses within a period of 0.1 to 1000 ns, the electric pulses have a pulse duration of 5 ns to 50000 ns, and the electric pulses, upon reaching the target voltage, have an electric field strength of 0.5 kV/cm to 100 kV/cm.
